# EUROPÄISCHE PATENTSCHRIFT

(11) **EP 2 266 619 B1**
(45) Veröffentlichungstag und Bekanntmachung des Hinweises auf die Patenterteilung: **13.05.2015**
(21) Anmeldenummer: 08873197.1
(22) Anmeldetag: 04.03.2008
(51) Int. Cl.: A61K 45/06, A61K 31/66, A61K 31/685, A61K 36/28, A61P 1/16, A23L 1/308, A23L 1/30

(54) **PHARMAZEUTISCHE ZUSAMMENSETZUNG AUF BASIS EINES LEBERSCHÜTZENDEN MITTELS UND EINES PRÄBIOTISCHEN MITTELS, IHRE HERSTELLUNG UND VERWENDUNG**
PHARMACEUTICAL COMPOSITION BASED ON HEPATOPROTECTOR AND PREBIOTIC, THE PRODUCTION AND USE THEREOF
COMPOSITION PHARMACEUTIQUE À BASE D'HÉPATOPROTECTEUR ET DE PRÉBIOTIQUE, FABRICATION ET UTILISATION DE CELLE-CI

(43) Veröffentlichungstag der Anmeldung: 29.12.2010
(73) Patentinhaber: Dikovskiy, Aleksander Vladimirovich, Moscow 123182 (RU)
(72) Erfinder: RUDOY, Boris Anatolievich, Moscow 109518 (RU); DOROZHKO, Oleg Valentinovich, Moscow 115446 (RU)
(74) Vertreter: Jeck, Anton
(86) Internationale Anmeldenummer: PCT/RU2008/000122
(87) Internationale Veröffentlichungsnummer: WO 2009/110816

(56) Entgegenhaltungen:
- EP-A2- 0 795 604
- DE-A1-102004 054 133
- RU-C1- 2 205 637
- RU-C1- 2 310 463
- KRUEGER K J ET AL: "Nutritional supplements and alternative medicine", CURRENT OPINION IN GASTROENTEROLOGY, GOWER ACADEMIC JOURNALS, LONDON, GB, Bd. 20, Nr. 2, 1. März 2003 (2003-03-01), Seiten 130-138, XP009131865, ISSN: 0267-1379
- NEYRINCK AUDREY M ET AL: "Kupffer cell activity is involved in the hepatoprotective effect of dietary oligofructose in rats with endotoxic shock", JOURNAL OF NUTRITION, Bd. 134, Nr. 5, Mai 2004 (2004-05), Seiten 1124-1129, XP002640031, ISSN: 0022-3166
- REGISTR LEKARSTVENNYKH SREDSTV ROSSII 2006, pages 976, 505 - 829, 895
- KOMPANIYA EVARAL. GEPATRIN (CAPSULY), [Online] July 2006, Retrieved from the Internet: <URL:http://www.evalar.ru/ru/catalogue/chan ge/?action=details> [retrieved on 2008-11-06]
- INKITIN I.G. ET AL.: 'Dyufalak (laktyloza) v lechenyn disbioza kishechnika pri nealkogolnom steatogepatite.' KLINICHESKIE PERSPEKTIVY GASTROENTEROLOGIYA I GEPATOLOGII vol. 1, 2002, pages 24 - 29

## Beschreibung

Die Erfindung betrifft eine pharmazeutische Zusammensetzung auf Basis von leberschützendem Arzneimittel und Präbiotikum zur Anwendung nach dem Oberbegriff des Anspruchs 1, und ihre Herstellung.

Die Erfindung ist in der Medizin und insbesondere in der Hepatologie und der Pharmakologie einsetzbar. Sie kann zur Herstellung und Anwendung einer pharmazeutischen Zusammensetzung auf der Basis von leberschützendem Arzneimittel ("hepatoprotector") und Präbiotikum (prebiotic) zur Behandlung und Vorbeugung von Lebererkrankungen aus folgender Gruppe von Krankheiten benutzt werden: Gallensteinleiden, Fettleber und nichtalkoholische Steatohepatitis, primäre biliäre Leberzirrhose, Gallenblasencholesterose, sowie arzneimittelbedingte und toxische Leberschädigung.

Die Zeitnähe des Problems hängt mit der Zunahme der Häufigkeit und des Schweregrades der Leberkrankheiten zusammen. Die Leber ist das Hauptorgan, wenn es um die Entgiftung von exogenen Giften geht. Die Ursachen für die Steigerung der Leberkrankheitsraten sind auf einen äußerst ungünstigen Umweltzustand in den meisten Regionen der Welt zurückzuführen.

Die Einwirkung von ökologischen Faktoren hängt ihrerseits und direkt mit einer Immunitätsschwächung bei der Bevölkerung zusammen. Dies verursacht ein beachtliches Wachstum von Infektionskrankheiten der Leber und vor allem von Virushepatitis.

Die Ansteckungsquelle bei einer Virushepatitis ist ein bereits von der Krankheit betroffener Mensch. Der Infektionsübertragungsweg ist entweder fäkal-oral oder parenteral je nach der Virusart: A, B, C, D, G, E. Die Empfindlichkeit der Bevölkerung gegenüber dieser Infektion ist hoch.

Unabhängig von der Eintrittspforte der Krankheit gelangt der Virus letztendlich in die Leber. Hier übt er eine direkte toxische Wirkung auf die Leberzellen aus. Diese Giftwirkung erfolgt darüber hinaus grundsätzlich gleichzeitig mit einer immunvermittelten Schädigung der Zellmembranen. Bei allen Formen der Virushepatitis ist eine der häufigen und schwierigsten Komplikationen die Störung der normalen Prozesse der Gallenbildung und -absonderung. Das ist ein so genanntes cholestatisches Syndrom, welches von Gelbsucht begleitet ist. Dieses Syndrom zeigt sich am häufigsten bei Virushepatitis A (HAV) - der sog. «enteralen» Virushepatitis - und bei Virushepatitis E, wo die Rate der Gelbsuchtformen 100 % beträgt.

Bei schweren Formen einer akuten Virushepatitis und bei Rezidiven einer chronischen Hepatitis ist die Störung der Struktur und der funktionellen Aktivität der biliären Gänge eine der Ursachen der Entwicklung von schwierigen Komplikationen der biliären Leberzirrhose.

Außer der Virushepatitis hängt ein großer Anteil der Lebererkrankungen mit einer Einwirkung von Nahrungsmittelgiften zusammen (Alkohol, andere toxische und Giftstoffe, verschiedene Arzneimittel).

Eine der frühesten pathologischen Komplikationen bei einer toxischen Leberaffektion ist eine Steatohepatitis. Steatohepatitis ist die Folge der Störung der positiven Bilanz zwischen dem Eintritt der Fette in den Körper und ihrem Metabolismus.

Es sei betont, dass die Störung der normalen Prozesse der Gallenbildung und ihrer biliären Passage nur eine der vielen verbreiteten Wirkungsfolgen einer großen Dosis vieler Arzneimittel ist (Antibiotika, p-Aminobenzolsulfonamide, Chlorpromazin, Blocker der Histaminrezeptoren und der östrogenen Hormone sowie Zytostatika).

In den letzten zehn Jahren wird immer häufiger die so genannte autoimmune Hepatitis diagnostiziert. Diese ist die Folge einer tiefgreifenden Störung im zellgebundenen Immunsystem. Eine der schwierigsten Folgeerscheinungen davon ist eine primäre biliäre Leberzirrhose.

Eine der markantesten Form der Störung der Prozesse der Gallenbildung und Gallenabsonderung ist das Gallensteinleiden (Cholelithiasis). Dabei ist eine überflüssige Gallenansammlung (Gallenstauung) in der Gallenblase mit nachfolgender Steinbildung (Cholelithe oder Gallensteine) zu beobachten.

Bei allen oben erwähnten Lebererkrankungen ist eine der wichtigen ätiologischen und pathogenetischen Faktoren die Störung der normalen Prozesse des Gallensäurestoffwechsels. Der Gallensäurestoffwechsel ist einer der wichtigsten Faktoren einer richtigen Verdauung.

Die Gallensäure wird in der Leber aus Cholesterin gebildet (s. Hofmann A.F. Bile acid secretion, bile flow and biliary lipid secretion in humans. Hepatologi. 1990; 12; 17S; Meier P.J. The bile salt secretory polarity of hepatocytes. J. Hepatol. 1989; 9: 124).

Zu den Hauptgallensäuren, die in der menschlichen Galle erkannt werden, zählen Cholsäuren (3a-, 7a-, 12a-Trioxy-5b-cholansäure), Chenodesoxycholsäure, Desoxycholsäuren (3a-, 12a-Dioxy-5b-cholansäure). Darüber hinaus sind in der Galle viel kleinere Mengen von Stereoisomere der Chol- und der Desoxycholsäuren in Form von Allocholsäure, Ursodesoxycholsäure und Lithocholsäure (3a-Manoxy-5b-cholansäure) festzustellen.

Die Cholsäure und die Chenodesoxycholsäure zählen zu den so genannten primären Gallensäuren. Sie werden in der Leber bei Cholesterinoxidation gebildet. Die Desoxycholsäure und die Lithocholsäure werden aus den primären Gallensäuren im Darm unter dem Einfluss der Fermente von Mikroorganismen der Darmflora gebildet.

Das Mengenverhältnis der Cholsäure, der Chenodesoxycholsäure und der Desoxycholsäure in der Galle beträgt im Normalfall 1 : 1 : 0,6.

In der Blasengalle sind die Gallensäuren meistens in Form von Paarlingen - den Konjugaten - vorhanden. Die Gallensäuren werden im Darm und meistens im Krummdarm ins Blut eingesaugt. Mit dem Blut kommen die Gallensäuren wieder in die Leber zurück und werden wieder in der Galle sezerniert. Das ist die so genannte portal-biliäre Gallensäurezirkulation. Deswegen entfallen 85 bis 90 % der gesamten in der Galle enthaltenen Gallensäuremengen auf die im Darm absorbierten Gallensäuren.

Die portal-biliäre Gallensäurezirkulation trägt dazu bei, dass die Konjugate der Gallensäure dank ihrer Löslichkeit in Wasser leicht in den Darm eingesaugt werden. Dabei werden 10 - 15 % der gesamten Gallensäuremenge im Darm unter Einwirkung der Fermente der Mikroorganismen der Darmflora gespalten. Die Produkte ihres Abbaus werden mit dem Kot abgeführt.

Die Gallensäuren emulgieren die Fette und sorgen somit für die Absorption der unlöslichen Fettsäuren und Cholesterin sowie der Vitamine B, K, E und der Kalksalze in den Dünndarm.

Darüber hinaus weisen die Gallensäuren eine sehr ausgeprägte gallentreibende Wirkung auf. Sie stimulieren die Darmmotorik und haben dazu noch eine bakteriostatische und entzündungshemmende Wirkung.

Unter Berücksichtigung des oben Dargelegten ist der eventuelle Bestandteil des Verfahrens zur Behandlung und Vorbeugung vieler pathologischer Leberzustände die Anwendung von Gallensäurepräparaten und vor allem der Ursodesoxycholsäure.

Die Ursodesoxycholsäure ist eine tertiäre Gallensäure. Sie wurde zum ersten Mal in der Galle eines chinesischen Bären 1902 entdeckt. Die Ursodesoxycholsäure ist in der Medizin im Laufe von mehreren Jahrhunderten eingesetzt. Im alten China wurde ausgetrocknete Bärengalle bei der Behandlung von Magen-, Darm- und Leberkrankheiten benutzt. Die Ursodesoxycholsäure wird unter Wirkung von Bakterienenzymen aus einer 7-Keto-lithocholsäure gebildet, welche in die Leber aus dem Dünndarm kommt.

Dabei sind die chemischen Formeln der Ursodesoxycholsäure und der hydrophoben Chenodesoxycholsäure identisch (C₂₄H₄O₄).

Die Anwendung der Ursodesoxycholsäure zur Behandlung insbesondere der Leberkrankheiten bedingt eine dosisabhängige Veränderung des oben genannten Gallensäurenverhältnis: Die Ursodesoxycholsäure wird zum Hauptbestandteil der Galle, während der Gehalt an Chenodesoxycholsäure und an anderen Gallensäuren abnimmt.

Die geringere Ansammlung der Ursodesoxycholsäure in der Galle ist bei den mit Leberkrankheiten betroffenen Kranken zu beobachten. Das kann mit einer Verminderung der Absorption infolge der Abnahme der Bildung der endogenen Mizellen aus den Gallensäuren in der Duodenalgalle oder mit einer Reduktion der Sekretion der eigentlichen Gallensäuren zusammenhängen.

Die Ursodesoxycholsäure und die Lithocholsäure werden in der Galle des Menschen wie bereits erwähnt in nur sehr geringen Mengen (0,1 % - 5 %) entdeckt.

Trotz der guten Absorptionsfähigkeit der Ursodesoxycholsäure im Darm bleibt ihr Spiegel im Blutplasma verhältnismäßig klein wegen der Leber-Clearance, weil in der Leber eine wirksame Konjugation der Ursodesoxycholsäure mit Glyzin, Taurin, Glukuronsäure und Sulfat zustande kommt.

Die Ursodesoxycholsäure übt in der Galle eine umfassende Einwirkung auf Cholesterin aus.

Die Cholesterinabsorption im Darm, seine Synthese in der Leber und die Sekretion in die Galle nimmt ab. Jedoch wird der Cholesterinspiegel im Blut unter der Wirkung der Ursodesoxycholsäure nicht wesentlich vermindert.

Die Ursodesoxycholsäure und ihre im Dünndarm, in den Distalabschnitten des Dünndarms und im Dickdarm nicht absorbierten Konjugate werden durch die indigenen Bakterien metabolisiert.

Die Ursodesoxycholsäure wird im Darm aufgespaltet und in Lithocholsäure dehydriert. Die Menge der Lithocholsäure ist in der menschlichen Galle sehr gering. Sie wird im Dünndarm unter dem Einfluss der Mikroflora bei der Verwertung vieler Fette gebildet. Aus dem Dünndarm kommt die Lithocholsäure in den Dickdarm und den Fettdarm. Hier wird sie teilweise absorbiert und gelangt in die Leber.

In der Leber wird die Lithocholsäure mit Sulfatanionen und danach mit Glyzin und Taurin gebunden und wird auf diese Weise in die Galle abgesondert. Ihre Derivate werden im Darm nur gering aufgenommen und mit dem Stuhl ausgeschieden.

Dieser Vorgang ist ein wirksamer Mechanismus für die Eliminierung der toxischen Lithocholsäure aus dem Körper.

Die Chenodesoxycholsäure bedingt eine Aktivitätsminderung der 3-Hydroxy-3-methylglutarylkoenzym A-Reduktase. Das ist ein Ferment, welches an der Cholesterinsynthese teilnimmt und zur Verminderung der Absorption von Cholesterin im Darm beiträgt. Das führt eine Verhältnisänderung der Gallensäuren und Cholesterin herbei, so dass die Chenodesoxycholsäure unter allen Gallensäuren dominiert.

Die genannten Mechanismen bestimmen die Nutzung der Chenodesoxycholsäure bei der Auflösung der Gallensteine, die vorwiegend aus Cholesterin bestehen.

Die Desoxycholsäure ist die Gallensäure, welche im Darm des Menschen unter dem Einfluss der Fermente der Darmflora gebildet wird. Sie wird in das Blut angesaugt und durch die Leber als Bestandteil der Galle sezerniert.

Es wird angenommen, dass das hydrophobe Salz der Desoxycholsäure ein Bindeglied zwischen der gestörten Darmmotorik und der Lithogenität der Galle sein kann. Die Hauptgallensäuren des Menschen sind die Cholsäure und die Chenodesoxycholsäure. Das sind primäre Gallensäuren, die in der Leber aus dem Cholesterin synthetisiert werden.

Die sekundäre Desoxycholsäure entsteht in den Distalabschnitten des Dünndarms und im Dickdarm aus der Cholsäure unter dem Einfluss der Fermente der Darmflora und zwar der bakteriellen 7-α-Dehydroxylase. Die Desoxycholsäure wird aus dem Darm teilweise absorbiert und in die Rezirkulation der Gallensäuren nach ihrer Konjugation mit Taurin oder Glyzin in der Leber absorbiert.

Die verlängerte Verweildauer im Darm verstärkt die Bildung der Desoxycholsäure infolge des bakteriellen Stoffwechsels während die Verkürzung dieser Verweilzeit die umgekehrte Wirkung bedingt.

Als Ergebnis variiert die Menge der Desoxycholsäure innerhalb eines ziemlich großen Bereich von 10 bis 30 % von dem Gesamtpool der Gallensäuren.

In der letzten Zeit wurde nachgewiesen, dass die Menge der gram - positiven anaeroben Bakterien und ihre 7-α-Dehydroxylase bedingte Aktivität im Blinddarm bei den von Gallensteinleiden betroffenen Patienten gegenüber den gesunden Patienten vergrößert ist.

Dabei wurde ein korrelativer Zusammenhang zwischen dem verlangsamten Transit über den Darm, dem vergrößerten Anteil an Desoxycholsäure, der Übersättigung der Galle mit Cholesterin und der Steinbildung festgestellt. Es wird angenommen, dass die Desoxycholsäure zur Lithogenität der Galle und der Steinbildung beiträgt, indem die Verweildauer über den Darm verlängert wird. Das erhöht seinerseits die Absorption von Cholesterin und gemäß dem Mechanismus der positiven Rückverbindung ist die Bildung der Desoxycholsäure selbst gefördert.

Darüber hinaus kann die Desoxycholsäure die Sekretion von Cholesterin in die Galle verstärken, indem sie die Leberzellenmembran, wo sich Cholesterin in Sphingomyelin-Domänen befindet, beansprucht. Sie kann auch eine Kristallisation von Cholesterin in der Galle verstärken, indem sie die Bläschen mit Cholesterin destabilisiert.

In der Gallenblase kommen die Gallensäuren vorwiegend in Form von Paarlingen - den Konjugaten - vor. Infolge der Konjugation der Gallensäuren mit Aminosäure Glycin wird Glykocholsäure oder Glykochenodesoxycholsäure gebildet. Bei der Konjugation der Gallensäuren mit Taurin (C₂H₇O₃N₅, Abbauprodukt der Aminosäure Cystein) werden Taurocholsäure oder Taurodesoxycholsäure gebildet.

Die Konjugation der Gallensäuren umfasst die Stufen der Bildung von KoA-der Gallensäurenestere - und der Verbindung des Moleküls der Gallensäure mit Glycin oder Taurin mittels der Amidbindung unter Beteiligung von dem Lysosomenzym der Acyltransferase. Das Verhältnis der Glycin- und Taurinkonjugate der Gallensäuren in der Galle beträgt im Durchschnitt 3:1 und kann je nach Zusammensetzung der Nahrung und dem Hormonzustand des Körpers variieren.

Somit ist die Störung des Gallensäurewechsels einer der wichtigsten pathogenetischen Faktoren der Entwicklung einer ganzen Reihe von Leberkrankheiten.

Aus dem Patent RU 2002123352, 27.03.2004, ist ein Verfahren zur Behandlung der oben genannten Leberkrankheiten bekannt, welches die Anwendung der Ursodesoxycholsäure-Präparate in Form der Mono- oder Komplextherapie vorsieht.

Es ist auch ein Verfahren zur Behandlung der Leberkrankheiten mittels der Anwendung einer Komplextherapie mit Chenodesoxycholsäure-Präparaten bekannt (s. Verzeichnis der Arzneimittel von Russland. Arzneimittelhandbuch. Herausgegeben von G.L. Vyshkovsky. M.: «RLS-2006», 2005, S. 895 - 896).

Jedoch lässt sich der therapeutische Effekt gemäß den genannten Verfahren der Behandlung nur nach einer ziemlich langen Zeit (von einigen bis zu 6 - 12 Monaten) der Anwendung der Heilmittel spüren. Es bedarf des Öfteren im Grunde genommen einer lebenslangen Einnahme dieser Heilmittel.

Das hängt im Wesentlichen damit zusammen, dass die Anwendung der Gallensäurepräparate als Monotherapie einen solchen wichtigen pathogenetischen Faktor wie der Darmdysbiose und die dadurch bedingten umfassenden Systemstörungen des Stoffwechsels nicht völlig beseitigen kann.

Als leberschützende Arzneimittel (hepatoprotectors) werden seit langem Substanzen mit einer anderen Struktur und mit anderen Wirkungsmechanismen benutzt. Jedoch wird ihre Zugehörigkeit zu den tatsächlichen leberschützenden Arzneimitteln durch viele Fachleute bestritten.

Dazu zählt des Öfteren zum Beispiel die Klasse der so genannten essentiellen Phospholipide.

Phospholipide, oder Phosphoglyceride, stellen hochspezifische Lipide dar und sind wichtige grundsätzliche Komponenten der Zellmembrane und der Membrane der Struktureinheiten von Zellen, z. B. wie Mitochondrien. Sie können als "essentielle" (unentbehrliche) Elemente für das Wachstum, die Entwicklung und das ordnungsgemäße Funktionieren aller somatischen Zellen bezeichnet werden. Ergänzend zu ihrer Rolle im Aufbau der Zellmembrane kann gesagt werden, dass Phospholipide wichtige Bestandteile der Lipoproteine, der "Lungensurfaktante" und der Galle sind. Sie nehmen in der Funktion des Nervensystems, in den Enzymreaktionen der Membranen teil und spielen eine wichtige Rolle im Stoffwechsel und in den Oxydationsvorgängen. Die Phospholipide sind Bestandteil der Lipoproteine und beeinflussen als solche die Cholesterinwerte im Blut.

Die in Thrombozyten eingebetteten Phospholipide nehmen an den Vorgängen der Blutgerinnung teil. Das zeigt im Endeffekt ihren Einfluss auf die Schutzfunktionen des Bluts und zeugt von der Hämodynamik im Körper der Säugetiere und des Menschen. Die chemische Struktur der Phospholipide, ihre diphyle Natur, das Vorhandensein von geladenen Gruppen legt die Einzigartigkeit ihrer physiologischen Eigenschaften fest.

Die Hauptfunktion der Phospholipide ist die Bildung der zweifachen Lipideschicht in den Zellmembranen. Die Struktur und die Funktion der Zellmembran hat eine sehr große Bedeutung für die Gesundheit des Menschen. Das Gefühl des allgemeinen Unwohlseins, die Funktionsstörung und verschiedene Krankheiten lassen sich in vielen Fällen durch eine Membranschädigung bzw. durch ihre Unstabilität erklären. Durch die Einführung der Phospholipide können die Membranfunktionen beeinflusst werden, welche mit den Membranproteinen zusammenhängen. In manchen Fällen können diese Funktionen sogar bis zu einem bestimmten Maß verbessert werden. Manchmal können sogar die gestörten Funktionen völlig korrigiert werden.

Die essentiellen Phospholipide dringen meistens in die Leberzellen ein. Sie betten sich in die Membrane der Hepatozyte ein, normalisieren die Leberfunktionen, den Lipiden- und Proteinenmetabolismus, tragen zur Aktivierung und zum Schutz der phospholipidenabhängigen Enzymsysteme bei und verbessern die Entgiftungsfunktion der Leber. Sie regenerieren die Zellenstruktur der Leber, verbessern die Regenerationsvorgänge und verlangsamen die Bildung des Bindegewebes in der Leber.

Das Arzneimittel vermindert den Energieaufwand in der Leber, setzt die Neutralfette und das Cholesterin in leicht metabolisierbare Formen um und stabilisiert die physikalischen und die chemischen Eigenschaften der Leber.

Die essentiellen Phospholipide normalisieren indirekt die Verdauung sowohl der Fette als auch der festen Nahrungsmittel im Darm, und zwar dank der Wiederherstellung der Leberzellenstruktur. Das bedingt eine Normalisierung der Gallenbildung und Gallenabsonderung (K.G. Gurevich. Essentielle Phospholipide bei der Behandlung der Leberkrankheiten. Klinische Qualitätspraxis. 2002, Heft 4, S. 108-111).

Ein anderer Vertreter der Gruppe der Heilmittel mit leberschützenden Eigenschaften ist ein Pflanzenextrakt von Mariendisteln als Hauptwirkstoff und der Bestandteil von Silymarin. Dieses Heilmittel hat eine leberschützende, regenerierende und Desintoxikationswirkung.

Diese Arznei neutralisiert die freien Radikale in der Leber, verhindert die Zerstörung der Zellenstrukturen, stimuliert auf eine spezifische Weise die RNS- Polymerase und aktiviert die Synthese der strukturellen und der funktionellen Proteine und Phospholipide in den beschädigten Hepatozyten. Sie stabilisiert die Zellmembrane, verhindert den Verlust der intrazellulären Komponenten (Transaminasen) und beschleunigt die Regeneration der Leberzellen. Sie verlangsamt das Eindringen von manchen giftigen und lebergefährdenden Stoffen in die Zelle (z. B. das Gift des grünen Knollenblätterpilzes).

Klinische Pharmakologie: Verbesserung des allgemeinen Zustandes der mit Leberkrankheiten betroffenen Patienten, Verminderung der subjektiven Beschwerde (Schwäche, Schweregefühl im rechten Unterrippenbereich, Appetitverlust, Erbrechen, Hautjucken), Normalisation der Laborwerte: Aktivität der Transaminasen, der Gamma- Glutamintransferase, der alkalischen Phosphatase, der Bilirubin-Werte. Bei Dauereinnahme wird das Leben der Kranken mit Leberzirrhose verlängert.

Einige Aminosäuren oder ihre Abkömmlinge werden oft auch den leberschützenden Arzneimitteln zugewiesen.

Das bekannteste davon ist Ademetionine (Verzeichnis der Arzneimittel von Russland. Arzneimittelhandbuch. Herausgegeben von G.L. Vyshkovsky. M.: «RLS-2006», 2005, S. 51).

Dieses Präparat ergänzt den Ademetioninemangel und stimuliert seine Erzeugung im Körper und vor allem in der Leber und im Gehirn. Das Molekül von S-Adenosil- L-Methionine (Ademetionine) gibt die Methylgruppe in den Methylierungsreaktionen der Phospholipide der Zellmembranen von Proteinen, Hormonen, Neurotransmitter usw. ab (Transmethylierung). Es ist der Vorgänger einer physiologischen Thiolverbindungen: Cystein, Taurin, Glutathion (zur Sicherstellung der Redoxmechanismen der Zellenentgiftung), KoA usw. in den Transsulfatierungsreaktionen. Nach der Decarboxylierung nimmt dieses Präparat an den Vorgängen der Aminopropylierung als Vorgänger der Polyamine teil: Putreszin (Stimulator der Zellenregeneration und der Proliferation der Hepatozyte), Spermidin und Musculamin, die der Struktur der Ribosome angehören. Das Präparat hat eine anti-cholestatische Wirkung. Es ist bei der intralobulären Form der Cholestase (d.h. bei der Störung der Synthese und des Flusses der Galle) hilfsreich. Die anti-cholestatische Wirkung ist durch die Beweglichkeit und die Polarisation der Hepatozytmembrane infolge der Stimulation der Synthese von Phosphatidylcholine in diesen Membranen bedingt. Das verbessert die Funktion der mit den Hepatozytmembranen assoziierten Systeme zum Gallensäurentransport und trägt zur Passage der Gallensäuren in das ableitende Gallensystem bei. Das Präparat stimuliert die Entgiftung der Gallensäuren, erhöht den Gehalt an konjugierten und sulfatierten Gallensäuren in den Hepatozyten. Die Konjugation mit Taurin erhöht die Lösbarkeit der Gallensäuren und ihre Ableitung aus dem Hepatozyt. Das Sulfatieren ermöglicht eine Elimination durch die Nieren, erleichtert die Passage durch die Leberzellenmembran und die Abführung mit der Galle. Außerdem schützen die sulfatierten Gallensäuren die Leberzellenmembrane gegen die toxische Wirkung der nichtsulfatierten Gallensäuren (diese sind mit hohen Konzentrationen in den Hepatozyten bei intrahepatischer Cholestase vorhanden).

Bei Patienten mit diffusen Leberkrankheiten (Zirrhose, Hepatitis) mit dem intrahepatischen Cholestase- Syndrom vermindert diese Arznei das Hautjucken und die Ausgeprägtheit der Veränderungen der biochemischen Werte darunter auch des direkten Bilirubins, der Aktivität von alkalischen Phosphatase (AP), der Aminotransferasen usw.

Während der Behandlung verschwand das asthenische Syndrom bei 54 % der Patienten; bei 46 % der Kranken nahm seine Intensität ab. Die antiastenische, anti-cholestatische und leberschützende Wirkung blieb im Laufe von 3 Monaten nach der Beendigung der Behandlung erhalten. Die Wirksamkeit bei den durch die Einnahme von lebervergiftenden Arzneimitteln (Paracetamol u.a.m.) bedingten Hepatopathien ist nachgewiesen. Bei der Behandlung der Patienten nach dem Opiummissbrauch mit begleitenden Leberschädigungen wurde eine Regression der klinischen Erscheinungen von Abstinenz, die Verbesserung des Funktionszustandes der Leber und der Vorgänge der Mikrosomie-Oxidation sowie eine stimmungshebende Wirkung beobachtet.

Anwendung: intrahepatische Cholestase; Leberaffektionen, darunter toxische, alkoholbedingte, virusbedingte, arzneimittelbedingte (Antibiotika, antiblastomatöse Mittel, Tuberkulostatika, virentötende Präparate, trizyklischen Antidepressiva, orale Kontrazeptiva); zirrhotische und vorzirrhotische Zustände; Enzephalopathie, darunter auch Enzephalopathie mit Leberinsuffizienz (alkoholbedingte u.a.m.); depressives Syndrom und Entziehungserscheinungen.

Außer dem unmittelbaren Einfluss auf das Lebergewebe weist Ademetionin einige zusätzliche pharmakologische Effekte auf, z. B. antidepressive Wirkung (sie entwickelt sich in der 1. Woche der Behandlung und wird in der 2. Woche der Behandlung stabilisiert). Darüber hinaus wird dieses Präparat erfahrungsgemäß bei Osteoarthritis angewendet. Das wird durch eine Minderung des Schmerzsyndroms und eine Stimulation der Synthese von Proteoglykanen und eine teilweise Regeneration des Knorpelgewebes begleitet.

Jedoch ergibt die Anwendung der essentiellen Phospholipide bei der Behandlung der Leberkrankheiten genau wie die Anwendung von anderen leberschützenden Arzneimittel normalerweise keine vollständige Genesung. Nach der Aufhebung werden oft Rezidive oder akute Erscheinungen der Leberkrankheiten beobachtet, darunter deswegen, weil die Störung der Darmbiozönose bestehen bleibt. Die Darmbiozönose ist bei vielen Erkrankungen der Organe vom Magen-Darm-Kanal und der Leber meistens sehr beharrlich.

Zurzeit gewinnt in der Gastroenterologie ein Konzept der Einheit (der Zusammengehörigkeit) aller Prozesse, die bei den Pathologien des Magen-Darm-Kanals zustande kommen, immer mehr Anerkennung. Im Rahmen dieses Konzepts ist einer der wichtigsten Bestandteile dieses normalen Zustands die Normalisierung der Dickdarmmikroflora.

Die Mikroflora des Magen-Darm-Kanals (MDK) und die Leber wirken ununterbrochen bei den Vorgängen der Entgiftung des Organismus zusammen. Die Mikrobiota als Bestandteil des Biofilms kommt als erstes in Kontakt und bei den nachfolgenden metabolischen Reaktionen mit allen Substanzen, die mit den Lebensmitteln, Wasser oder Frischluft in den Körper gelangen. Die Mikrobiota setzt die chemischen Stoffe in ungiftige Endprodukte bzw. in Zwischenverbindungen um, die sich in der Leber leicht zerlegen und danach aus dem Körper entfernen lassen.

Der Organismus hat zwei Entgiftungshauptorgane: Die Leber sorgt für den Schutz des Organismus mittels Oxidationsreaktionen. Die Mikroflora des Verdauungstraktes nutzt dafür die hydrolytischen Reduktionsprozesse. Die Störung der Zusammenwirkung dieser Systeme verursacht gegenseitige funktionelle und strukturelle Veränderungen sowohl in diesen Organen als auch im ganzen Körper.

Deswegen kann die enterohepatische Zirkulation von unterschiedlichen organischen und anorganischen Verbindungen ohne Übertreibung als einer der grundlegenden homöostatischen Mechanismen bezeichnet werden. Die Minderung der Entgiftungsfunktion der Mikroflora von MDK bei der durch verschiedene Pathogene (Medikamente, Nahrungsmittel, Stress usw.) bedingten Dysbiose erhöht die Belastung der enzymatischen Systeme der Leber und trägt bei beistimmten Bedingungen zu metabolischen und strukturellen Veränderungen bei.

Beim Ungleichgewicht der Mikroumweltbedingungen des Verdauungstraktes führt die Vergrößerung des Anteils der potentiell pathogenen gram-negativen Bakterien zu einer beachtlichen Ansammlung von Endotoxinen im Darmlumen.

Die letzteren dringen durch die Schleimhaut des Darmes in das lokale Kreislaufsystem, und danach durch die Pfortader in die Leber ein und verursachen Hepatozytverletzungen oder ermöglichen ungünstige Einwirkungen anderer Giftstoffe. 90 % aller Endotoxine werden optional durch die anaeroben gram-negativen Bakterien freigesetzt. Die Endotoxinen beschädigen die Zellmembranen, stören den Ionentransport, verursachen die Fragmentation der Nukleinsäuren, induzieren die Bildung der Produkte der Freiradikaloxydation, regen die Apoptose an usw. (N. M. Gracheva und andere. Hylak forte bei der Komplexbehandlung der Patienten mit akuten Darminfektionen und chronischen Krankheiten des Magen-Darm-Kanals mit Dysbakteriose-Erscheinungen. Consilium medicum. 2004. Heft 1. S. 31 - 34).

Folglich ist einer der möglichen Wege der Wiederherstellung nach den Störungen in der Gesamtheit der Wechselwirkungen von Mikrobiota und Leber die Bekämpfung der Darmdysbiose.

Aus der Schrift von V.F. Demin und anderen ("Erfahrung bei der Anwendung von der bio-phytogenen Korrektur bei Kindern mit Dysbiose. Probleme der modernen Kinderheilkunde, 2003, Heft 3, Band. 2, S. 33 - 36)" ist ein Verfahren zur Normalisierung der Darmmikroflora dank der Verabreichung (per os) von Probiotika bekannt. Es handelt sich dabei um lebendige Bakterien solcher Arten und Gattungen, die im Normfall den Dickdarm des Menschen und anderer Säugetiere besiedeln. Jedoch ergibt der Einsatz von Probiotika in Form der Monotherapie keine beständigen Ergebnisse infolge der «Fremdartigkeit» der von außerhalb eingeführten Bakterienstämme sowie infolge ihrer ziemlich schnellen (3 - 5 Tage) Entfernung nach dem Abbruch der Einnahme des Heilmittels.

Dieser Mangel liegt bei anderen Präparaten, die zur Korrektur der Störungen der Darmmikrobiota eingesetzt werden - den so genannten Präbiotika -, nicht vor.

Zu den Präbiotika zählen insbesondere viele Oligosaccharide. Sie werden durch den menschlichen Körper nicht verwertet, weil es im Darm keine eigenen Enzyme gibt, die solche Zuckerarten aufspalten können. Zu den verdaulichen Oligosacchariden zählen unter anderem: Fruktooligosaccharide, Maltooligosaccharide, Galaktooligosaccharide, Inulin, Laktulose und einige andere Oligosaccharide, die als Präbiotika eingesetzt werden können (S.A. Sheveleva Probiotika, Präbiotika und probiotische Produkte. Stand von Heute. Ernährungsangelegenheiten, 1999, Heft 2, S. 33 - 39; Shoaf L. et al. Prebiotic galactooligosaccharides reduce adherence of Enteropathogenic Escherichia coli to tissue culture cells. Infect Immun. 2006 Sept. 18. Abstr.).

Der chemischen Struktur nach sind Fruktooligosaccharide Oligofruktosaccharide, in denen die Reste der β-, D-Fruktofuranose untereineinder durch die β-2, 1-glykosidische Bindungen verbunden sind und an einem Ende der Kette ein Reststück von α-Glukose haben, welches mit der Fruktose durch die Bindung α-1, 2 verbunden ist. Sie können als Saccharose-Abkömmlinge betrachtet werden, an deren Fruktoseteil 1 bis 3 Fruktofuranose Reste durch die Bindungen β-2, 1 angehängt sind. Die Hauptkomponenten der Fruktooligosaccharide sind 1-Kestose (GF₂), Nistose (GF₃) und 1F-Fruktofuranosilnistose (GF₄).

Die Fruktooligosaccharide verfügen über eine ausgeprägte präbiotische Wirkung. Sie werden in den oberen Abschnitten des MDK nicht ausgenutzt, hemmen das Wachstum der faulen Mikroflora, fördern die Normalisierung des Blutdrucks und den Lipidespiegel im Blut, verbessern die Aufnahme von Calcium und Magnesium, erhöhen die Immunität, haben eine gute Wirkung bei Verstopfungen und Eiterungsprozessen und verhindern den Dickdarmkrebs.

Wie alle Präbiotika werden die Fruktooligosaccharide keiner Hydrolyse durch die Fermente von MDK ausgesetzt. Sie werden in den Dünndarm nicht eingesaugt und, nachdem sie in den Dickdarm unverändert gelangen, dienen sie als eine selektive Grundlage für ein Wachstum der normalen Mikroflora.

Die Laktulose ist ein Disaccharid, welcher aus Galaktose und Fruktose besteht (4-0-D-Galaktopyranosil-D-Fruktose). Unter den natürlichen Bedingungen kann die Laktulose in kleinen Mengen bei einer Milcherhitzung bis über 100°C gebildet werden. Die Laktulose ist im Wasser gut lösbar und ist ca. 1,5 bis 2-fach süßer als Laktose.

Die präbiotische Wirkung der Laktulose zeigt sich durch eine Vergrößerung des Umfangs des Dickdarmes, eine Verminderung von pH sowie durch eine Minderung des Ammoniakgehalts im Dickdarm und eine Erhöhung des Gehalts an kurzkettigen Fettsäuren, insbesondere an Propionsäure (ZDUNCZYK Z et al. Physiological effects of lactulose and inulin in the caecum of rats. Arch Anim Nutr. - 2004. - Vol. 58(1). - P. 89 - 98).

Der Einfluss der Laktulose auf die Darmmikroflora durch eine Erhöhung der Menge von Bifidusbakterien unter Steigerung der Aktivität der mirkobiellen β-Galaktosidasen ist auch bekannt (BOUCHNIC Y. et al. Prospective, randomized Parallel-group trial to evaluate the effects of lactulose and polyethylene glycol-4000 on colonic flora in chronic idiopathic constipation. Aliment. Pharmacol. Ther. - 2004. - Vol. 19(8). - P. 889-899).

Während Laktulose ein Präbiotikum ist, wird sie bis jetzt in der Therapie meistens und sogar ausschließlich als ein weiches und wirksames Abführmittel benutzt. Die abführenden Eigenschaften der Laktulose hängen direkt mit dem präbiotischen Effekt zusammen und sind durch eine Vergrößerung des Umfangs des Inhalts vom Dickdarm (ca. um 30 %) bedingt, weil die Bakterienpopulation vermehrt wird.

So ist z. B. ein Verfahren zur Normalisierung des gestörten Zustandes der Darmmikroflora bekannt, welches den Einsatz von Präbiotika und insbesondere von verdaulichem Oligosaccharid (Laktulose, Fruktooligosaccharid u.a.m.) einschließt (JP 2003-155242, 27.05.2003).

In der Fachliteratur gibt es ziemlich wenig Informationen über die Erfahrungen mit der Anwendung von Präbiotika zur Behandlung der Leberkrankheiten.

Gemäß den veröffentlichten Angaben (I.G. Nikitin u.a.m. Duphalac (Laktulose) bei der Behandlung der Darmdysbiose bei nicht alkoholischer Steatohepatitis. Klinische Aussichten der Gastroenterologie, Hepatologie, Koloproktologie, 2002, Heft 1, S. 24 - 29; V.S. Saveliev. Lipiden-Distress-Syndrom in der Chirurgie. Informationsblatt Der Militärmedizinischen Akademie Russlands. 1999, Heft 1, S. 36 - 39) zeigt sich ihre Anwendung an für sich als Monopräparate bei den Versuchen der Behandlung der Leberkrankheiten nur wenig effektiv, weil die Zellenschädigungen und folglich die Funktionen des betroffenen Lebergewebes in diesem Fall nicht vollständig beseitigt werden.

Der therapeutische Effekt der Anwendung von nur Präbiotika erscheint erst nach einer ziemlich langer Zeit. In diesem Fall gelingt es nicht, eine vollständige Wiederherstellung des gestörten Lipiden- und insbesondere Cholesterinwechsels zu erreichen. Darüber hinaus wurde bei den beschriebenen Forschungen die Laktulose in großen Mengen (von ca. 30 ml des konzentrierten Sirups pro Person) eingesetzt. Dies ist jedoch nicht als präbiotische sondern als abführende Wirkung bekannt.

Solche große Dosen an Laktulose werden in der Hepatologie normalerweise vorwiegend zur Abschwächung der Symptome einer hepatischen Enzephalopathie benutzt, d. h. bei einer palliativen symptomatischen Zustandserleichterung bei Kranken, die bereits so gut wie unheilbar sind (Zirrhose im letzten Stadium). In solchen Fällen ist die Normalisierung der Darmmikroflora nicht imstande, einen mehr oder weniger langfristigen Effekt sicherzustellen.

Deswegen wird bei den Therapien von verschiedenen Krankheiten immer mehr eine umfassende Anwendung von Arzneimitteln unterschiedlicher Gruppen z. B. mit immunomodulierender Wirkung und Antibiotika, gallentreibenden Mitteln usw. vorgezogen.

In diesem Zusammenhang ist z. B. ein Verfahren zur Behandlung der chronischen nicht kalkulösen Cholezystitis mit Dysbioseerscheinungen dank der Anwendung der Kombination des leberschützenden Arzneimittels (Glutargin) und Eubiotikum (Bifiform) bekannt (UA 70018,15.09.2004).

Jedoch ist der therapeutische Effekt dieser Kombination wegen der oben genannten Einschränkungen, die mit der Anwendung der Probiotika zusammenhängen, kurzfristig.

Es ist auch ein Verfahren zur Behandlung der Leberkrankheiten mit cholestatischem Syndrom bekannt, indem die Kombination des leberschützenden Arzneimittels pflanzlicher Herkunft (Silibum marianum - Extrakt) mit dem probiotischen Stamm von Lactobacillus bulgarinii und einigen anderen Stoffen verwendet wird (BG 108250U, 30.04.2005).

Jedoch ist auch in diesem Fall der erreichte positive therapeutische Effekt relativ kurzfristig.

Das bekannte Verfahren zur Korrektur des Savelievs Lipiden-Distress-Syndroms unter Einsatz einer Komplextherapie umfasst ein leberschützendes Arzneimittel pflanzlicher Herkunft - Hepabene und das Metabolit-Probiotika Hylak forte (V.A. Petukhov. Funktionsstörung der Leber und Dysbiose beim Lipiden-Distress-Syndrom. RMZh, 2002, Heft 10, Band 4, S. 158 - 160).

Jedoch sind in dieser Gegenüberstellung weder das Behandlungsschema mit den genannten Medikamenten noch ihre Dosen bzw. Verhältnisse angegeben. Es ist auch keine Zusammenwirkung der benutzten Heilmittel beschrieben.

Aus dem EA 5166 (30.12.2004) ist auch die Anwendung von einem kombinierten Präparat zur Behandlung der Leberkrankheiten bekannt, welches als Hauptwirkungskomponente eine alkalische Sphingomyelinase und als ergänzende Mittel verschiedene Substanzen darunter auch Probiotika (Lactobacillus acidophilus, Lactobacillus brevis, Lactobacillus buchneri, Lactobacillus casei, Lactobacillus catenaforme), Präbiotika und Ursodesoxycholsäure aufweist.

Die Hauptrolle wird im oben genannten Stand der Technik der Sphingomyelinase (lysosomes Enzym) zugewiesen. Die Sphingomyelinase ist zur Vorbeugung bzw. zur Behandlung von verschiedenen Erkrankungen benutzt, die folgender Gruppe angehören: Störung der Dünndarmaktivität, bösartige Tumore, Störungen des Immunsystems, Entzündungen und Desquamation der Schleimhaut des Dünndarms, Zustände, die mit den Störungen der Cholesterol-Synthese zusammenhängen, Störungen der Einsaugfähigkeit des Dünndarms und allergische Dünndarmerkrankungen.

Dabei gehören zu den Hilfsstoffen in der genannten pharmazeutischen Zusammensetzung: Probiotika (Lactobacillus acidophilus, Lactobacillus brevis, Lactobacilus casei u.a.m.), Abkömmlinge der Gallensäuren - Ursodesoxycholsäure und das Präbiotikum - Laktulose.

Jedoch enthält die Patentschrift keine Hinweise auf die Rolle dieser Hilfsstoffe bei der Behandlung von Leberkrankheiten. Sie offenbart keine wissenschaftliche Begründung ihrer Angehörigkeit zu dieser Zusammensetzung.

Dieser Anwendung der pharmazeutischen Zusammensetzung zur Behandlung der genannten Erkrankungen ist derselbe Hauptmangel eigen - eine zu kurze Wirkungszeit, die durch den exogenen probiotischen Stamm bedingt ist. Dabei werden die in der Zusammensetzung vorhandenen präbiotischen Komponenten im großen Maße durch den oben eingeführten exogenen Stamm verwertet.

Die große Menge von Komponenten mit verschiedenen Wirkungen ermöglicht es nicht, die Rolle dieser Komponenten und ihren therapeutischen Effekt richtig einzuschätzen. Sie schließt auch einen gegenseitigen Antagonismus dieser Effekte nicht aus. Das vergrößert die Wahrscheinlichkeit der individuellen Variationen bei der Reaktion auf die gleichzeitige Einführung von so vielen Präparaten.

Der nächste Stand der Technik gegenüber der Erfindung ist ein Agens, welches die Leberfunktionen verbessert und als Spender der Methylgruppe fungiert. Es enthält einen schwerverdaulichen Oligosaccharid, welches seinerseits Galaktose enthält, und wird als Produkt einer funktionellen Ernährung benutzt (JP 2003-155242, 27.05.2003).

Dabei ist als Spender der Methylgruppe eine Gruppe von Aminosäuren gewählt, die S-Adenosilmethionin umfasst. Der galaktosehaltige Oligosaccharid ist aus einer Gruppe gewählt, die unter anderem Laktulose oder Galaktooligosaccharid einschließt.

Jedoch betrachten die Erfinder in der oben genannten Patentschrift überhaupt keine präbiotischen Effekte der Zusammensetzung. Die Rolle der Oligosaccharide im vorgeschlagenen Agens ist (vermutlich) durch die Erfinder nur auf die Beseitigung der durch den Ammoniak induzierten hepatoenzephalopathische Intoxikation zurückgeführt.

Somit sehen die Erfinder gemäß der genannten Patentschrift keinen Zusammenhang zwischen der den Lipidenwechsel normalisierenden Wirkung der Verbindungen, die als Spender der Methylgruppen fungieren, und der Normalisierung des Zustandes der Darm-Mikrobiozönose. Dieser Zustand wird in den angeführten Beispielen der genannten Patentschrift überhaupt nicht erwähnt.

Das fehlende Verständnis eines ununterbrochenen Zusammenhangs zwischen dem Zustand der Darm-Mikrobiozönose und den Reaktionen des Lipidstoffwechsels, der Beteiligung der Vertreter normaler Mikroflora am Durchbrechen des Teufelskreises einer enteropathogenen Gallensäurerezirkulation ermöglicht es nicht, die richtige Zusammensetzung der Komponenten je nach dem Störungsgrad dieser wichtigsten Bestandteile des Stoffwechselvorgangs zu wählen und die Adäquatheit und die Effektivität der durchgeführten Therapie der Leberkrankheiten im erforderlichen Maße zu überwachen.

Da die Rolle der ersten Komponente der Zusammensetzung nur auf die Funktion des Spenders der Methylgruppe begrenzt ist, lassen die Erfinder andere Mechanismen der Störung des Lipidstoffwechsels unbegründet unbeachtet. Es geht dabei insbesondere um den Cholesterinstoffwechsel. Solche Mechanismen spielen inzwischen eine sehr wichtige Rolle bei der Entwicklung von vielen Leberkrankheiten.

Der Unterschied der Erfindung gegenüber dem nächsten Stand der Technik ist wie folgt:
- die Lebererkrankungen sind genau festgelegt und unterscheiden sich gegenüber dem nächsten Stand der Technik. Die pharmazeutische Zusammensetzung ist ausgerechnet zur Behandlung von festgelegten Lebererkrankungen entwickelt,
- die entwickelte pharmazeutische Zusammensetzung umfasst ein leberschützendes Arzneimittel und ein Präbiotikum, welches aus einer streng begrenzten Anzahl der Vertreter dieser Gruppen der Heilmittel gewählt ist,
- es ist die Zweckmäßigkeit einer gleichzeitigen Einführung des leberschützenden Arzneimittels und eines Präbiotikums in einer pharmazeutischen Zusammensetzung unter Berücksichtigung ihres synergistischen Einflusses aufeinander nachgewiesen,
- es ist das Verhältnis des leberschützenden Arzneimittel und des Präbiotikums in der pharmazeutischen Zusammensetzung festgelegt.

Es ist Aufgabe der Erfindung, einen wesentlichen positiven Effekt wie eine Beschleunigung der Normalisierung des Gesundheitszustandes und die Minderung der Ausgeprägtheit der Symptome der Erkrankung bei einer Komplexbehandlung von einzelnen Leberkrankheiten zu erreichen.

Die gestellte Aufgabe wird durch die Merkmale des Anspruchs 1 gelöst.

Dieses Ziel ist durch die Anwendung eines kombinierten (Komplex-) Heilmittels mit einer Mischung von leberschützendem Arzneimittel und präbiotischem Stoff, nämlich von den im Darm nicht verdaulichen Oligosacchariden, bei der Behandlung der Leberkrankheiten erreicht.

Das durch diese Erfindung erreichte technische Ergebnis ist eine möglichst schnelle Wiederherstellung der Leberfunktionen und die Vorbeugung der Rezidive der Erkrankungen durch einen Wiederaufbau des Cholesterinstoffwechsels und der Darmbiozönose, welche durch eine synergische Zusammenwirkung vom leberschützenden Arzneimittel (hepatoprotector) und dem Präbiotikum bedingt ist. Das bedingt auch eine Vorbeugung gegen Nebenwirkungen des leberschützenden Arzneimittels.

Als leberschützendes Arzneimittel sind Gallensäuren / Salze der Gallensäuren benutzt, die aus folgender Gruppe gewählt sind: Glykocholsäure, Glykochenodesoxycholsäure, Taurocholsäure, Taurodesoxycholsäure, Ursodesoxycholsäure, Chenodesoxycholsäure und essentielle Phospholipide.

Das Vorhandensein des leberschützenden Arzneimittels und des Präbiotikums in der pharmazeutischen Zusammensetzung stellt eine ausgeprägte und andauernde therapeutische Wirkung sicher dank einer synergistischen Wirkung des leberschützenden Arzneimittels und des Präbiotikums.

Die synergistische Wirkung des leberschützenden Arzneimittels, z. B. der Ursodesoxycholsäure, und des Präbiotikums ist dadurch bedingt, dass die Ursodesoxycholsäure die Verdauung nicht nur der Fette sondern auch der festen Nahrung im Darm dank der Erhöhung der Synthese der Gallensäuren und der eigentlichen Ursodesoxycholsäure in der Leber normalisiert. Das trägt zur Normalisierung der Darmmikroflora bei. Das Präbiotikum selbst fördert die Normalisierung der Mikroflora durch eine Stimulation des Wachstums der residenten Stämme. Das bringt die Erscheinung von immunostimulierenden Effekten und anderer Effekte der Normoflora mit sich. Folglich sind die Verdauungsprozesse und die Entgiftung der exogenen Gifte der Mikroflora verbessert. Das vermindert auch eine metabolische Belastung der Leber und trägt zur Normalisierung des Fettsäuren- und Cholesterinstoffwechsels bei. Dies übt auch eine stabilisierende Wirkung auf alle Körperzellen einschließlich der Hepatozyte aus.

Somit ermöglicht die Verbindung der Gallensäuren oder Gallensäurensalze mit den im Dünndarm verdaulichen Oligosacchariden aus der Gruppe Laktulose oder Fruktooligosaccharide, Maltooligosaccharide, Galaktooligosaccharide, Inulin in einer pharmazeutischen Zusammensetzung, die Funktion der Hepatozyte und der ganzen Leber durch Normalisierung der Darmbiozönose wiederherzustellen. Das stellt seinerseits eine Stabilisation des erreichten therapeutischen Ergebnisses für eine lange Zeit sicher.

Bekanntlich ist bei Lebererkrankungen und Gallensteinleiden mit vorwiegend Cholesterinsteinen im Blut des Patienten ein erhöhter Gehalt an toxischen Produkten zu beobachten, die aus dem Dickdarm ins Blut kommen. Es handelt sich dabei insbesondere um Ammoniak. Das ist eine Stickstoffverbindung, die während der bakteriellen Proteinzersetzung im Dickdarm durch proteolytische Mikroflora gebildet ist. Dies vergrößert die toxische Belastung der Leber.

Somit trägt die Wiederherstellung der Mikroflora im Darm indirekt zur Verminderung der toxischen Belastung der Leber und zur Zunahme der Ursodesoxycholsäure in der Leber bei. Dies fördert seinerseits den Wiederaufbau der Gallenzusammensetzung und nämlich der Zunahme der Gallensäuren dank der verminderten Cholesterinsynthese durch die Leber. Dadurch sind die Rezidive der Erkrankung und insbesondere die Gallensteinleiden mit vorwiegend Cholesterinsteinen vermieden.

Unter Berücksichtigung des oben Dargelegten verstärken die aktiven Komponenten der erfindungsgemäßen pharmazeutischen Zusammensetzung gegenseitig die vorhandenen Heileigenschaften jeder der Komponenten.

Die erfindungsgemäße pharmazeutische Zusammensetzung kann wird definiert durch Anspruch 1.

Die erfindungsgemäße pharmazeutische Zusammensetzung ist in Form von Tabletten (Pillen) (mit oder ohne Hülle), Granula, Globulus, Pulver, Kapseln, Suspensionen, Emulsionen oder Gele hergestellt.

Dabei enthält die erfindungsgemäße pharmazeutische Zusammensetzung zusätzlich die in der pharmazeutischen Produktion allgemein verwendete Hilfsstoffe wie mikrokristalline Cellulose, Laktose, Maisstärke, Steife, Hydroxypropylmethylcellulose, Carboxymethylcellutose, Oxypropylmethylcellulose, Oxypropylcellulose, ihre pharmazeutisch verträglichen Salze, Ludipress, Calciumstearat, Magnesiumstearat, Polysorbat, Polyvinylpyrrolidon, Polyethylenglycol, Talk, Titandioxid oder Siliciumdioxid.

Die erfindungsgemäße Zusammensetzung ist mittels Vermengung der zugehörigen Zutaten hergestellt. Dazu zählen sowohl aktive Komponenten (leberschützendes Arzneimittel und Präbiotikum nach Anspruch 1, als auch Hilfsstoffe, die aus folgender Gruppe gewählt sind: mikrokristalline Cellulose, Laktose, Maisstärke, Steife, Hydroxypropylmethylcellulose, Carboxymethylcellulose, Oxypropylmethylcellulose, Oxypropylcellulose, ihre pharmazeutisch verträglichen Salze, Ludipress, Calciumstearat, Magnesiumstearat, Polysorbat, Polyvinylpyrrolidon, Polyethylenglycol, Talk, Titan-dioxid oder Siliciumdioxid.

Die erfindungsgemäße pharmazeutische Zusammensetzung ist oral mit viel Wasser im Laufe von 1,5 bis 3 Monaten eingenommen.

Die erfindungsgemäße pharmazeutische Zusammensetzung ist zur Behandlung von Kranken mit Leberkrankheiten aus folgender Gruppe anwendbar: Gallensteinleiden mit vorwiegend Cholesterinsteinen, alkoholische und nichtalkoholische Steatohepatitis, primäre biliäre Leberzirrhose, Gallenblasencholesterose, arzneimittelbedingte und toxische Leberschädigung. Diese Zusammensetzung ermöglicht es, innerhalb einer relativ kurzen Zeit (von 6 bis 12 Wochen) eine andauernde Remission der Krankheit zu erreichen.

Dabei beträgt die therapeutische Effektivität 89 % bis 95 %.

Die erfindungsgemäße pharmazeutische Zusammensetzung hat keine Gegenanzeigen. Sie ist zur Behandlung von Patienten mit den oben genannten Leberkrankheiten einsetzbar, darunter im Hintergrund schwerer Begleitkrankheiten (mit Ausnahme von den späten Stadien der Leberzirrhose, der malignen Neubildungen des Magen-Darm-Kanals oder anderer Organe), unabhängig vom Alter des Patienten.

Die erfindungsgemäße Zusammensetzung weist keine wesentlichen Nebenwirkungen auf, da ihre aktiven Bestandteile in kleinen und mittleren Einzelheildosen und zwar innerhalb eines kurzen Zeitraums angewendet werden.

Die erfindungsgemäßen Ausführungsformen der Zusammensetzung zeichnen sich durch niedrige Selbstkosten aus und sind dementsprechend für alle Sozialgruppen von Patienten zugänglich.

Die Behandlung der Patienten erfolgt ambulant. Sie bedarf keiner strikten oder halbstrikten Bettruhe. Deswegen können die Patienten ihre gewöhnliche Lebensweise pflegen.

Die erfindungsgemäße pharmazeutische Zusammensetzung ist nicht nur zur Behandlung sondern auch zur Vorbeugung der Rezidive von Leberkrankheiten aus folgender Gruppe erfolgreich verwendbar: Gallensteinleiden vorwiegend mit Cholesterinsteinen, alkoholische und nichtalkoholische Steatohepatitis, primäre biliäre Leberzirrhose, Gallenblasencholesterose, arzneimittelbedingte und toxische Leberschädigung. Das ist dank dem Wiederaufbau der Struktur und der Funktion sowohl der Hepatozyte als auch der gesamten Leber möglich, was durch die Normalisierung der Darmmikroflora bedingt ist.

Die Einwirkung der erfindungsgemäßen pharmakologischen Zusammensetzung in bestimmter Dosis sieht eine allmähliche und ansteigende Verstärkung der Heilwirkung und den Beitritt der neuen Regulationsebenen der Homöostase: subzellulare, intrazelluläre, Gewebe-, Organ-, System- und Organismusebene vor, und zwar dank dem Wiederaufbau des Lipidstoffwechsels, insbesondere des Cholesterinstoffwechsels, welcher durch die Normalisierung der Mikrodarmbiozönose bedingt ist.

Während der ambulanten Behandlung ist dem Kranken empfohlen, bestimmte Tagesregime einzuhalten, mindestens dreimalige Verpflegung am Tag während der gesamten Behandlungsdauer; es wird vom Verzehr von Alkohol, Fett- und scharfen Nahrungsmitteln sowie von der Einnahme anderer Heilmittel abgeraten. Während der Behandlung ist eine Hungerkur und eine schwere körperliche Arbeit verboten.

Die endgültige Diagnose ist aufgrund zusätzlicher Untersuchungen (US-Kontrolle der Leber oder die R-graphie der ableitenden Gallenwege) und der Laboruntersuchungen des Bluts (Biochemie: Cholesterin und seine Fraktionen, Bilirubin und seine Fraktionen, alkalische Phosphatase, Alanine Aminotransferase, Aspartate Aminotransferase, Blutkörperchensenkungsgeschwindigkeit u.a.m.) gestellt.

Die Erfinder haben 60 Patienten untersucht. Dabei sind bei allen Patienten klinische Symptome der Leberaffektionen mit einem oder anderen Ausprägungsgrad erkannt: Gelbfärbung der Haut und der Sklera, Hautjucken, unangenehme Empfindungen oder Schweregefühl im rechten Unterrippenbereich, Dyspepsionserscheinungen: Übelkeit, Appetitlosigkeit, Erbrechen, Schwäche, Trägheit, Veränderung der Urinfarbe (dunklere Farbe) und des Stuhls (Abführung oder Durchfall).

Alle Patienten sind sowohl ambulant als auch stationär behandelt. Dabei sind verschiedene Heilmittel verwendet worden.

Die meisten Kranken (37 von 60) hatten Begleitkrankheiten: Chronische Gastroduodenitis; Herz-Kreislaufsystemkrankheiten wie Hochdruckkrankheit, ischämische Herzkrankheit; Lungenkrankheiten: Pneumosklerose, Bronchialasthma u.a.m. Außerdem sind bei 85 % der Patienten Begleitstörungen des Mikrobiozönosezustandes des Dickdarmes erkannt werden. Alle Kranken aus den Versuchsgruppen (50) sind mit der pharmazeutischen Zusammensetzung gemäß der ausgearbeiteten Regime der Einführung behandelt: 3 Mal täglich beim Essen im Laufe von 1,5 - 3,0 Monaten.

Gegen Ende der 2. Woche sind Entgiftungs- und Synthesefunktionen der Leber im Hintergrund der Darmbiozönose bei allen 50 Kranken trotz des Charakters von Leberaffektionen festgestellt worden.

Alle Kranken stellten eine Minderung der unangenehmen Gefühle im rechten Unterrippenbereich und eine Verbesserung des Allgemeinbefindens bereits am 5. Tag fest. Am Anfang der 2. Woche ist bei allen Kranken subjektiv ein Verschwinden der Dyspepsie-Störungen, eine Wiederherstellung des Appetits, eine Normalisierung von Urin und Stuhl, ein Verschwinden von Hautjucken und eine ursprüngliche Hautfarbe im Hintergrund einer beachtlichen Verbesserung des Allgemeinzustands und -Stimmung feststellbar. Dabei ist parallel dazu eine Minderung oder ein Verschwinden der Symptome einer Begleitpathologie erkannt. Die Biochemie-Analyse des Bluts ergab am Ende der 3. Woche die Normalisierung aller Biochemiewerte, die die Leberfunktion und den Lipidstoffwechsel charakterisieren.

### Beispiele für die Ausführung des Verfahrens:

### 1. Kranker I., 46 Jahre.

Bei der Einlieferung ins Krankenhaus Beschwerden über ziehende Schmerzen im rechten Unterrippenbereich, die in die rechte Schulter ausstrahlen. Diese Schmerzen entstehen 3 - 4 Stunden nach dem Verzehr von Fettspeisen oder nach reichlichem Essen, oder nach körperlichen Belastungen; allgemeine Schwäche, Appetitlosigkeit, Übelkeit, periodisches Erbrechen, bitterer Geschmack im Mund, dünnflüssiger Stuhl oder manchmal Durchfall, Hautjucken, Veränderung der Harnfarbe (dunklere Farbe) und der Stuhlfarbe (heller).

Anamnese: Gallensteinleiden im Laufe von 10 Jahren. Keine Operationen. Ambulante Behandlung ohne besonderen Effekt. Zustandesverschlechterung nach körperlicher Belastung.

Objektives Bild: Überernährung, Gewicht 75 kg, Wuchs. 167 cm, blasse Hautdecken, Kratzspur auf dem Rücken und auf dem Bauch. Gelbfärbung der Lederhaut der Augen.

Weicher Bauch, schmerzhaft in der Kerr-Stelle. Positive Symptome von Kerr, Phrenikus-Symptom und Murphy-Symptom. Leber am Rand des Rippenbogens. Vesikuläratmen in den Lungen. Frequenz der Atembewegungen: 18 pro Minute. Herzgrenzen sind innerhalb der Altersnorm. Die Töne sind mäßig bedämpft, regelmäßiger Rhythmus, Herzfrequenz 78 pro 1 Minute, arterieller Druck 140/85 mm Hg. Negatives Pasternazki-Merkmal an beiden Seiten.

Vorläufige Diagnose: Chronisches Gallensteinleiden in der Rezidivphase.

### Untersuchung:

Allgemeine Blutprobe: Hb 123 g/l; Erythrozyten (Er) 4,11*10¹²/l; Farbenindex (F. I.) 0,89; Leukozyte 4,0* 10⁹/l; stäbchenkernige Leukozyte (StK) 2 %; segmentkernige Leukozyte (SK) 46 %; eosinophile Leukozyte (EPh) 5 %; Lymphozyte (Lph) 45 %; Monozyte (M) 2 %; Blutkörperchensenkungsgeschwindigkeit 40 mm/Stunde.

Allgemeine Harnprüfung: relative Dichte 10¹⁶; kein Eiweiß und Glukose entdeckt; Leukozyte 0-1-3 im Blickfeld; Erythrozyte 0 im Blickfeld; Harnamylase 16,2 mgc/l.

Koprogramm: wenige Muskelfaser ohne Streifung; Fettsäuren mäßige Menge; nicht verdautes pflanzliches Zellgewebe (viele), Stärke; Einzelzellen.

Kotprobe - Dysbakteriose: Verminderung der Menge von Bifidus- und Laktobakterien, jeweils: 10⁵/g, 10⁶/g, durch die Zunahme der Pilze aus der Gattung Candida.

### Blutbiochemie:

- Bilirubin und seine Fraktionen: allgemeines Bilirubin (AB) - 22,8 µMol/l (N 3,4 - 20,5 µMol/l); direktes Bilirubin (DB) - 3,8 µMol/l (N - 0,85 - 3,4 µMol/l), indirektes Bilirubin (IB) - 11,7 µMol/l (N - 2,56 - 10,3 µMol/l);
- Thymolprobe (TP) - 12,1 Einheiten (N - 4 Einheiten), Aspartate Aminotransferase - 79 Einheiten (N - 60 Einheiten) Alanine Aminotransferase - 72 Einheiten (N - 50 Einheiten), Thymolprobe (TP) 1,7 Einheiten (N - 4 Einheiten), alkalische Phosphatase (AP) - 362 Einheiten (N - bis zu 295 Einheiten), Cholat-Cholesterin-Faktor 15,3; Zucker 3,5 mMol/l, (N - 4,4 - 6,6 mMol/l).
- Cholesterin und seine Fraktionen: allgemeines Cholesterin (ACh) - 5,5 mMol/l (N - 3,65
- 5,2 mMol/l), Cholesterin der hochdichten Lipoproteide
- 0,8 mMol/l (N - 0,9 - 1,9 mMol/l), Cholesterin der niederdichten Lipoproteide - 3,2 mMol/l (N - 1,91 - 2,6 mMol/l), Cholesterinfaktor der Atherogenität CFA 3,5 Bezugseinheiten (N - bis zu 3 Bezugseinheiten), Cholat-Cholesterin-Faktor 15,3 (N - bis 12).
- Proteinfraktionen: Gesamteiweiß 67 g/l (N 65 - 85 g/l); Albumine 34 g/l (N - 36 - 50 g/l).
- antinukleare Antikörper: Antimikrosomale Antikörper im Titer 1:10;
- Koagulogramm: Prothrombinindex 24 s - 79 %; Thrombinzeit 35 s; freies Heparin 12 s; Fibrinogen 2,2 g/l; fibrinolytische Aktivität > 240 Minuten.

Koprogramm: Dysbakteriose durch die Verminderung der Anzahl der Lakto- und der Bifidusbakterien: Laktobakterien (10⁵) (N >= 10⁷/g), Bifidusbakterien (10⁷) (N >= 10⁹).

R-Graphie der Leber und der ableitenden Gallenwege - indirekte Merkmale des Gallensteinleidens, keine Steine lassen sich kontrastieren.

### Empfehlung

US-Untersuchtung von Leber und Gallenblase.

US-Untersuchtung der Leber: chronische Cholezystitis, Cholesterinsteine: 0,9, 1,2, 1,5, 1,3 mm, glatte Kanten.

EKG - Sinusrhythmus, Merkmale der mäßigen Hypertrophie der linken Herzkammer. Arterieller Druck 150/85 mm Hg; Herzfrequenz 74 pro 1 Minute.

Endgültige Diagnose: Lipidstoffwechselstörung, Hypercholesterinämie. Chronisches Gallensteinleiden (Cholesterinsteine) in der Rezidivphase.

Behandlung: Anwendung der erfindungsgemäßen Zusammensetzung, deren Aktivsubstanzen leberschützendes Arzneimittel Ursodesoxycholsäure und Laktulose im Verhältnis 1 : 2 (Einzeldosis der Ursodesoxycholsäure beträgt 325 mg), Intussuszeption 3 mal täglich beim Essen im Laufe von 1,5 Monaten und gleichzeitig Diät Nr. 5. Wiederholte Untersuchung nach 1,5 Monaten:
US-Untersuchung: Einzelsteine mit Abmessungen von 1 und 2 mm.

Angaben der Laboruntersuchung - keine Pathologie festgestellt.

Empfehlung: Fortsetzung der Behandlung im Laufe von 3 Monaten.

### Nach 3 Monaten

US-Untersuchung: Merkmale der chronischen Cholezystitis, Keine Steine vorhanden.

Gutachten: chronische Cholezystitis in der Remissionsphase.

### 2. Kranker B. 45 Jahre.

Bei der Einlieferung ins Krankenhaus: Beschwerden über Appetitlosigkeit, Schwäche, Übelkeit, periodisches Erbrechen, ziehenden Schmerzen im rechten Unterrippenbereich nach vielem Essen oder nach fettigen Speisen.

Anamnese: Chronischer Alkoholismus. Primäre biliäre Leberzirrhose. Unregelmäßige Behandlung.

Objektives Bild: Unterernährung. Trockene heiße Haut. Eicht gelbliche Hautdecken, Gelbfärbung der Lederhaut der Augen.

Lungenklangschall über den Lungen. Schwaches Atmen, zerstreute trockene Rasselgeräusche über der gesamten Lungenoberfläche. Frequenz der Atembewegungen 20 pro 1 Minute.

Herzgrenzen sind nach links um 1,0 cm erweitert. Gedämpfte Töne. Regelmäßiger Rhythmus, Akzent des 2. Tons über der Aorta.

Weicher Bauch, rechter Leberrand tritt über den Rand des Rippenbogens um 2,0 cm hinaus, dichter Rand. Die Milz ist nicht vergrößert.

Zweifelhaftes Pasternazki-Merkmal.

### Vorläufige Diagnose: Primäre biliäre Leberzirrhose?

### Untersuchung:

Allgemeine Blutprobe: Hb 117 g/l; Erythrozyte (Er) 3,5*10¹²/l; Farbenindex (F. I.) 0,9; Leukozyte 4,0*10⁹/l; stäbchenkernige Leukozyte (StK) - 17 %, Leukozyte 6 %; segmentkernige Leukozyte (SK) 36 %; eosinophile Leukozyte (EPh) 5 %; Lymphozyte 35 %; Monozyte (M) 1 %; Blutkörperchensenkungsgeschwindigkeit 40 mm/Stunde. Allgemeine Harnprüfung: relative Dichte 10¹²; kein Eiweiß und Glukose entdeckt; Leukozyte 0-2-3 im Blickfeld; Erythrozyte 0-2 im Blickfeld; Harnamylase 14,7 mgc/l.

### Koprogramm: Muskelfaser ohne Streifung - nicht viel; Fettsäuren - mäßige Menge; pflanzliches Zellgewebe.

### Blutbiochemie:

- Bilirubin und seine Fraktionen: allgemeines Bilirubin (AB) - 28,4 µMol/l (N 3,4 - 20,5 µMol/l); direktes Bilirubin (DB) — 4,8 µMol/l (N — 0,85 — 3,4 µmo/l), indirektes Bilirubin (IB) — 15,0 µMol/l (N — 2,56 — 10,3 µMol/l); - Thymolprobe (TP) - 16,1 Einheiten (N - 4 Einheiten), Aspartate Aminotransferase - 90 Einheiten (N - 60 Einheiten) Alanine Aminotransferase - 74 Einheiten (N - 50 Einheiten), alkalische Phosphatase (AP) - 700 Einheiten (N - bis zu 295 Einheiten), Zucker 6,6 mMol/l, (N - 4,4 - 6,6 mMol/l).
- Cholesterin und seine Fraktionen: allgemeines Cholesterin (ACh) - 5,9 mMol/l (N - 3,65 - 5,2 mMol/l), Cholesterin der hochdichten Lipoproteide - 10,8 mMol/l (N - 0,9 - 1,9 mMol/l), Cholesterin der niederdichten Lipoproteide - 3,6 mMol/l (N - 1,91 - 2,6 mMol/l), Cholesterinfaktor der Atherogenität CFA 3,9 Bezugseinheiten (N - bis zu 3 Bezugseinheiten), Cholat-Cholesterin-Faktor 16,3 (N - bis 12); (Norm: bis zu 50);
- Proteinfraktionen: Gesamteiweiß 63 g/l (N 65-85 g/l); Albumine 34 g/l (N - 36-50 g/l).
- Antikörper: Antimikrosomale Antikörper im Titer 1 :45
- Koagulogramm: Prothrombinindex 24 S - 79 %; Thrombinzeit 31 S; freies Heparin 11 S; Fibrinogen 2,0 g/l; fibrinolytische Aktivität > 221 Minuten.

Kotprobe - Dysbakteriose: Verminderung der Menge der Lakto- und Bifidusbakterien: Laktobakterien (10⁴), Bifidusbakterien (10⁶).

R-Graphie der Leber und der ableitenden Gallenwege: Vergrößerung der Leber im rechten Leberlappen um 2,5 cm, deutliche Kanten, indirekte Merkmale der biliären Leberzirrhose.

Empfehlung: US-Untersuchtung von Leber und Gallenblase.

US-Untersuchung der Leber: Merkmale der geringen Fettinfiltration der Leber und der Gallenblasencholesterose. Das Pankreas ist nicht vergrößert. Die intrahepatisehen und die extrahepatischen Gallenwege sind nicht erweitert. Es wurden keine Merkmale der Portalhypertonie festgestellt.

Keine Portalhypertonie festgestellt.

Leberbiopsie: Erweiterte Portalwege sind durch Lymphozyte, Plasmazellen, Makrophage und eosinophile Leukozyte infiltriert. Unter den Infiltratzellen der Portalwege kommen ausgebildete Limphoidfollikel vor. Die Infiltrate werden in den Wänden mancher Limphoidfollikel intralobulären Gallengänge erkannt. Die Ganzheit der Basalmembran der Gallengänge ist teilweise beschädigt. Neben den betroffenen Gallengängen gibt es Granulome, die aus den Epithelioidzellen und den mehrkernigen Riesenzellen gebaut sind.

### Gutachten: biliäre Leberzirrhose

EKG: Sinusrhythmus, Merkmale der mäßigen Hypertrophie der linken Herzkammer. Arterieller Druck 150/85 mm Hg; Herzfrequenz 76 pro 1 Minute.

Endgültige Diagnose: Lipidstoffwechselstörung, Hypercholesterinämie. Primäre biliäre Leberzirrhose.

Behandlung: Anwendung der erfindungsgemäßen Zusammensetzung, deren Aktivsubstanzen das leberschützende Arzneimittel Chenodesoxycholsäure und der Fruktooligosaccharid im Verhältnis 1 : 250 (Einzeldosis der Chenodesoxycholsäure beträgt 250 mg) sind, Intussuszeption 3mal täglich beim Essen im Laufe von 1,5 Monaten im Hintergrund der Diät Nr. 5.

### Wiederholte Untersuchung nach 1,5 Monaten:

US-Untersuchung: positive Dynamik: Verminderung der Fettinfiltration der Leber.

Blutprobe: Abnahme der Hypercholesterinämie - 4,6 mMol/l, Cholesterin der hochdichten Lipoproteide -1,2 mMol/l, Cholat-Cholesterin-Faktor 13,2; Allgemeines Bilirubin - 21,5 µMol/l, Antimikrosomale Antikörper 1:30; Gesamteiweiß - 72 g/l, Albumine - 34 %, Blutzucker 5,3 mMol/l, alkalische Phosphatase - 301 Einheiten, Aspartate Aminotransferase - 70 Einheiten, Alanine Aminotransferase - 62 Einheiten, TP - 8,1 Einheiten.

Kotprobe für Dysbakteriose: Laktobakterien 10⁶, Bifidusbakterien - 10⁷.

Empfehlung: Fortsetzung der Behandlung im Laufe von 3 Monaten.

Nach 3 Monaten - Antimikrosomale Antikörper 1:15, Bifidusbakterien 10⁹/g, Laktobakterien 10⁷/g.

### Gutachten: primäre biliäre Leberzirrhose der 1 St. (beachtliche positive Dynamik)

### 3. Kranke F., 50 Jahre

Bei der Einlieferung ins Krankenhaus: Beschwerden über die ausgeprägte allgemeine Schwäche, Schweregefühl und ziehende Schmerzen im rechten Oberteil des Bauchs, die Schmerzen kommen ohne sichtbare Gründe, Appetitlosigkeit, Übelkeit, periodisches Erbrechen, bitterer Geschmack im Mund, dünnflüssiger Stuhl, manchmal Durchfall, Hautjucken, Veränderung der Harnfarbe (dunklere Farbe) und Stuhlfarbe (heller).

Anamnese: Zuckerkrankheit des II. Typs im Laufe von 15 Jahren. Die Patientin nimmt hypoglykanisches Präparat Bucarban ein. Die Patientin steht unter Dispensairekontrolle des Endokrinologen und des Bereichsinternist. Die Patientin wird regelmäßig stationär in der endokrinologischen Abteilung behandelt, jedoch ohne besondere Ergebnisse. Die letzte Verschlechterung führt auf die Virusinfektion (verzögerter Verlauf; Komplikationen: akute Bronchitis, Einnahme der antibakteriellen Präparate Zephalosporine) zurück.

Objektives Bild: Überernährung: Übergewichtigkeit der 3. Stufe, Körpermasse- Index (KMI) 34, blasse Hautdecken, Kratzspuren auf dem Bauch und an der Innenoberfläche der Hüften. Gelbfärbung der Lederhaut der Augen.

Die Abmessungen des Bauchs sind wesentlich vergrößert. Der Bauch ist weich, schmerzhaft in der Kerr-Stelle. Die Leber tritt aus dem Rand des Rippenbogens um 2,5 cm hervor. Dichter Rand. Die Milz ist nicht vergrößert.

Abgeschwächtes Vesikuläratmen (wegen Fettzellstoffes) in den Lungen. Frequenz der Atembewegungen 22 pro 1 Minute.

Die Herzgrenzen sind erweitert: nach rechts um 1,0 cm, nach links ha 1,5 cm. Gedämpfte Töne, regelmäßiger Rhythmus, weiches systolisches Herzgeräusch über der Herzspitze. Herzfrequenz 76 pro Minute, Arterieller Druck 160/85 mm Hg. Zweifelhaftes Pasternazki-Merkmal an beiden Seiten.

Vorläufige Diagnose: Fetthepatose (?) Zuckerkrankheit des II. Typs, Übergewichtigkeit der 3. St.

### Untersuchung:

Allgemeine Blutprobe: Hb 121 g/l; Erythrozyte (Er) - 4,15*10¹²/l; Farbenindex (F. I.) - 0,89; Leukozyte - 3,8*10⁹; stäbchenkernige Leukozyte (StK) - 7 %; segmentkernige Leukozyte (SK) - 40 %; eosinophile Leukozyte (EPh) - 5 %; Lymphozyte 45 %; Monozyte (M) 3 %; Blutkörperchensenkungsgeschwindigkeit 39 mm/Stunde. Blutglukose 6,8 mMol/l.

Allgemeine Harnprüfung: relative Dichte 10¹⁶; Eiweiß Spuren; Leukozyte 3-5 im Blickfeld; Erythrozyte 0 im Blickfeld; Harnamylase 16,2 mgc/l.

Koprogramm: Muskelfaser ohne Streifung - nicht viel; Fettsäuren - mäßige Menge; nicht verdautes pflanzliches Zellgewebe - viel; Stärke; Einzelzellen.

Kotprobe für Dysbakteriose: Verminderung der Menge von Bifidus- und Laktobakterien, 10⁵/g, 10⁶/g (jeweils) durch die Zunahme der Pilze der Gattung Candida.

### Blutbiochemie:

- Bilirubin und seine Fraktionen: allgemeines Bilirubin (AB) - 27,0 µMol/l (N 3,4 - 20,5 µMol/l); direktes Bilirubin (DB) - 3,6 µMol/l (N - 0,85 - 3,4 µMol/l), indirektes Bilirubin (IB) - 11,2 µMol/l (N - 2,56 - 10,3 µMol/l);
- Thymolprobe (TP) - 8,0 Einheiten (N - 4 Einheiten), Aspartate Aminotransferase - 69 Einheiten (N - 60 Einheiten) Alanine Aminotransferase
- 76 Einheiten (N - 50 Einheiten), Thymolprobe (TP) 1,7 Einheiten (N - 4 Einheiten), alkalische Phosphatase (AP) - 346 Einheiten (N - bis zu 295 Einheiten), Cholat-Cholesterin-Faktor 15,3; Zucker 6,9 mMol/l, (N - 4,4 - 6,6 mMol/l).
- Cholesterin und seine Fraktionen: allgemeines Cholesterin (ACh) - 5,9 mMol/l (N-3,65 - 5,2 mMol/l), Cholesterin der hochdichten Lipoproteide - 0,8 mMol/l (N- 0,9 - 1,9 mMol/l), Cholesterin der Lipoproteide mit niedriger Dichte - 3,6 mMol/l (N - 1,91 - 2,6 mMol/l), Cholesterinfaktor der Atherogenität CFA 3,8 Bezugseinheiten (N - bis zu 3 Bezugseinheiten), Cholat-Cholesterin-Faktor 16,3 (N - bis 12);
- Triglyzeride: 1,94 mMol/l (N - 0,45 - 1,82 mMol/l)

Proteinfraktionen: Gesamteiweiß 67 g/l (N 65-85 g/l); Albumine 38 g/l (N - 36-50 g/l). - Koagulogramm: Prothrombinindex 24 S - 79 %; Thrombinzeit 35 S; freies Heparin 12 S; Fibrinogen 2,2 g/l; fibrinolytische Aktivität > 240 Minuten.

Koprogramm: Dysbakteriose infolge der Verminderung der Anzahl der Lakto- und der Bifidusbakterien: Laktobakterien (10⁴) (N>= 10⁷/g), Bifidusbakterien (10⁶) (N>= 10⁹/g).

R-Graphie der Leber und der ableitenden Gallenwege: gleichmäßige Lebervergrößerung. Keine Steine vorhanden.

Empfehlung: US-Untersuchung der Leber und der Gallengänge.

US-Untersuchung der Leber: Hepatomegalie der 2. St. Das Pankreas ist nicht vergrößert.

Die intrahepatischen und die extrahepatischen Gallenwege sind nicht erweitert. Merkmale der portalen Hypertension.

Ösophagogastroduodenoskopie: Erweiterung der Speiseröhrenvenen b/3.

EKG - Sinusrhythmus, Merkmale der mäßigen Hypertrophie der linken Herzkammer, unvollständiger Schenkelblock rechts. Arterieller Druck 160/85 mm Hg; Herzfrequenz 74 pro 1 Minute.

Endgültige Diagnose: Lipidstoffwechselstörung, Triglyzeridemie. Fetthepatose. Zuckerkrankheit des II. Typs. Übergewichtigkeit der 3. St.

Behandlung: Anwendung der erfindungsgemäßen Zusammensetzung, deren Aktivsubstanzen das leberschützende Arzneimittel - essentielle Phospholipide - und Fruktooligosaccharide im Verhältnis 1 : 50 (Einzeldosis der essentiellen Phospholipide beträgt 50 mg) sind. Intussuszeption 3mal täglich beim Essen im Laufe von 1,5 Monaten im Hintergrund der Diät Nr. 5.

### Wiederholte Untersuchung nach 1,5 Monaten:

Die Patientin stellte die Verminderung des Hautjuckens fest (so gut wie kein Jucken). Erhöhte Aktivität. Gewichtsabnahme um 5 kg.

US-Untersuchung: Kleinere Abmessungen der Leber.

Gutachten: Fetthepatose, Hepatomegalie der 1. St.

Angaben der Laboruntersuchung: Verminderung des Triglyzeridegehalts - 1,82 mMol/l und des allgemeinen Cholesteringehalts - 5,0 mMol/l. Normalisierter Blutzucker - 4,9 mMol/l.

Kotprobe: Beachtliche Vermehrung der Lakto- und Bifidusbakterien -10⁷/g und 10⁹/g (jeweils). Es wurden keine Pilze vom Typ Candida erkannt.

Empfehlung: Fortsetzung der Behandlung im Laufe von 3 Monaten.

### Nach 3 Monaten:

US-Untersuchung: Merkmale der mäßigen Hepatomegalie. Blutprobe enthält keine Besonderheiten. Die Darmmikroflora wurde wiederaufgebaut: Laktobakterien 10⁷/g, Bifidusbakterien 10⁹/g.

Gutachten: Fetthepatose, Merkmale der mäßigen Hepatomegalie. Zuckerkrankheit der II. St., Kompensation. Übergewichtigkeit der 3 St.

### Beispiel 4.

### Prüfung der akuten Toxizität der Zusammensetzung

Die Zusammensetzung der Ursodesoxycholsäure und der Laktulose im Verhältnis 1 : 2 (Gruppe 1), Ursodesoxycholsäure und Fruktooligosaccharide im Verhältnis 1 : 50 (Gruppe 2), essentielle Phospholipide (Lezithin) und Galaktooligosaccharide im Verhältnis 1 : 30 (Gruppe 3), Ademetionine und Laktulose im Verhältnis 1 : 50 (Gruppe 4) wurde den Weißmäusen (rassenlos) mit Körpergewicht von 15 - 20 g oral verabreicht.

Die Kontrollgruppe (Gruppe 6) bekam die gleiche Menge der Stärkeaufschwemmung. Die Tiere wurden 96 Stunden lang beobachtet. Ihr allgemeiner Zustand (Aussehen, Aktivität, Regelmäßigkeit der Nahrungs- und Wasseraufnahme, Art und Natur der Exkremente) wurde regelmäßig erfasst.

| Mischung (Gruppe) | Max. Menge des verabreichten Präparats, g | Überlebensfähigkeit (lebendig / tot) | Aussehen | Nahrung | Beweglichkeit | Art und Natur der Exkremente |
|---|---|---|---|---|---|---|
| 1 | 2 | 6/0 | N | N | N | N |
| 2 | 2 | 6/0 | N | N | N | N |
| 3 | 2 | 6/0 | N | N | N | N |
| 4 | 2 | 6/0 | N | N | N | N |
| 5 | 2 | 6/0 | N | N | N | N |
| 6 | 2 | 6/0 | N | N | N | N |

Die Ergebnisse der Untersuchung sagen aus, dass die akute Toxizität jeder Zusammensetzung unter Versuchsbedingungen sich nicht erkennen lässt. Alle Mischungen gehören zur Klasse der schadstoffarmen Stoffe (die LD-50 Werte überschreiten 100 g/kg des Körpergewichts).

Somit kann die erfindungsgemäße pharmazeutische Zusammensetzung, die als Aktivsubstanzen leberschützendes Arzneimittel und Präbiotikum aus den im Darm nicht verdaulichen Oligosaccharide enthält, zur Anwendung unter klinischen Bedingungen zur Behandlung und Vorbeugung der Leberkrankheiten aus der Gruppe: Gallensteinleiden, Fetthepatose und nichtalkoholische Steatohepatitis, primäre biliäre Leberzirrhose, Gallenblasencholesterose, arzneimittelbedingte und toxische Leberschädigung empfohlen werden.

## Patentansprüche

1. Pharmazeutische Zusammensetzung zur Verwendung in der Behandlung und Vorbeugung der Rezidive von Leberkrankheiten, welche durch eine Störung des Lipid-Cholesterinstoffwechsel bedingt sind, wobei sie aus folgender Gruppe gewählt sind: Gallensteinleiden vorwiegend mit Cholesterinsteinen, alkoholische und nichtalkoholische Steatohepatitis, primäre biliäre Leberzirrhose, Gallenblasencholesterose, arzneimittelbedingte und toxische Leberschädigung, wobei die pharmazeutische Zusammensetzung innerlich angewendet ist und als Aktivsubstanzen ein leberschützendes Arzneimittel, welches aus folgender Gruppe gewählt ist: Cholsäuren, Chenodesoxycholsäuren, Desoxycholsäuren, Ursodesoxycholsäuren, Lithocholsäuren, Tauroursodesoxycholsäuren, Glykodesoxycho-Isäuren, Taurocholsäuren, Glykocholsäuren, und Lactulose als Präbiotikum aufweist, die in wirksamen Dosen gewählt sind.

2. Pharmazeutische Zusammensetzung zur Verwendung nach Anspruch 1,
**dadurch gekennzeichnet,**
**dass** sie innerlich als Nährungsergänzungsmittel angewendet sind.

3. Pharmazeutische Zusammensetzung zur Verwendung nach Anspruch 1 oder 2,
**dadurch gekennzeichnet,**
**dass** sie als leberschützendes Arzneimittel Gallensäure oder Gallensäurensalz und Präbiotikum im Verhältnis von 1 : 2 bis 1 : 250 nach der Reinsubstanzmasse aufweist.

4. Pharmazeutische Zusammensetzung zur Verwendung nach Anspruch 1,
**dadurch gekennzeichnet,**
**dass** sie als leberschützendes Arzneimittel essentielle Phospholipide aufweist, die aus folgender Gruppe gewählt sind: Phosphatidylcholin, Kephalin, Phosphatidyl inositol.

5. Pharmazeutische Zusammensetzung zur Verwendung nach Anspruch 1 oder 4,
**dadurch gekennzeichnet,**
**dass** sie als leberschützendes Arzneimittel essentielle Phospholipide und Präbiotikum im Verhältnis von 1 : 0,1 bis 1 : 100 nach der Reinsubstanzmasse aufweist.

6. Pharmazeutische Zusammensetzung zur Verwendung nach Anspruch 1,
**dadurch gekennzeichnet,**
**dass** sie zusätzlich mikrokristalline Zellulose, Laktose, Maisstärke, Steife, Hydroxypropylmethylzellulose, Karboxymethylzellulose, Oxypropylmethylzellulose, Oxypropylzellulose, ihre pharmazeutisch zulässige Salze, Ludipress, Kalziumstearat, Magnesiumstearat, Polysorbat, Polyvinylpyrrolidon, Polyäthylenglykol, Talk, Titandioxid oder Siliziumdioxid aufweist.

7. Pharmazeutische Zusammensetzung zur Verwendung nach Anspruch 1,
**dadurch gekennzeichnet,**
**dass** sie in Form von Tabletten, Granula, Globulus, Pulver, Kapseln, Suspensionen, Pasten, Sirupe, Emulsionen oder Gelen ausgebildet ist, welche für eine innerliche Anwendung in solchen Dosen vorgesehen sind, die den bekannten Dosen der leberschützenden Arzneimittel und Präbiotika, 2 - 3 mal täglich entsprechen.

8. Herstellung einer pharmazeutischen Zusammensetzung nach Anspruch 1 zur Behandlung von Leberkrankheiten, die aus folgender Gruppe gewählt sind: Gallensteinleiden vorwiegend mit Cholesterinsteinen, alkoholische und nichtalkoholische Steatohepatitis, primäre biliäre Leberzirrhose, Gallenblasencholesterose, arzneimittelbedingte und toxische Leberschädigung, wobei die in wirksamen Dosen genommenen aktiven Komponenten - leberschützendes Arzneimittel und Präbiotikum - mit mikrokristalliner Zellulose, Laktose, Maisstärke, Kartoffelstärke, Hydroxypropylmethylzellulose, Karboxymethylzellulose, Oxypropylmethylzellulose, Oxypropylzellulose, mit ihren pharmazeutisch zulässigen Salzen, Ludipress, Kalziumstearat, Magnesiumstearat, Polysorbat, Polyvinylpyrrolidon, Polyäthylenglykol, Talk, Titandioxid oder Siliziumdioxid vermengt sind.

## Claims

1. A pharmaceutical composition for use in the treatment and prevention of recurrences of liver diseases that are caused by a disturbance in lipid-cholesterol metabolism, the diseases being selected from the group comprising gallstone conditions predominantly involving cholesterol calculi, alcoholic and nonalcoholic steatohepatitis, primary biliary cirrhosis, gallbladder cholesterosis, and drug-induced and toxic liver damage, wherein the pharmaceutical composition is taken internally and has as its active ingredients a liver-protective medication selected from the group comprising cholic acids, chenodeoxycholic acids, deoxycholio acids, ursodeoxycholic acids, lithocholic acids, tauroursodeoxycholic acids, glycodeoxycholic acids, taurocholic acids, glycocholic acids, and lactulose as a prebiotic, which are selected in effective doses.

2. The pharmaceutical composition for use according to claim 1,
**characterized in that**
they are taken internally as nutrition supplements.

3. The pharmaceutical composition for use according to claim 1 or 2,
**characterized In that**
as the liver-protective medication, it has bile acid or bile salt and prebiotic in a ratio of 1 : 2 to 1 : 250 by mass of the pure substance.

4. The pharmaceutical composition for use according to claim 1,
**characterized in that**
as the liver-protective medication, it has essential phospholipids selected from the group comprising phosphatidyl choline, cephalin, and phosphatidyl inositol.

5. The pharmaceutical composition for use according to claim 1 or 4,
**characterized in that**
as the liver-protective medication, it has essential phospholipids and prebiotic in a ratio of 1 : 0.1 to 1 : 100 by mass of the pure substance,

6. The pharmaceutical composition for use according to claim 1,
**characterized in that**
it additionally has microcrystalline cellulose, lactose, cornstarch, stiffening, hydroxypropyl methylcellulose, carboxymethylcellulose, oxypropyl methylcellulose, oxypropyl cellulose, their pharmaceutically permissible salts, Ludipress, calcium stearate, magnesium stearate, polysorbate, polyvinylpyrrolidone, polyethylene glycol, talcum, titanium dioxide, or silicon dioxide.

7. The pharmaceutical composition for use according to claim 1,
**characterized in that**
it is embodied in the form of tablets, granules, pellets, powder, capsules, suspensions, pastes, syrups, emulsions, or gels, which are intended for internal use in doses, corresponding to the known dosages of liver-protective medications and preblot-Ics, 2 to 3 times per day.

8. Production of a pharmaceutical composition according to claim 1 for treatment of liver diseases selected from the group comprising gallstone conditions predominantly involving cholesterol calculi, alcoholic and nonalcoholic steatohepatitis, primary biliary cirrhosis, gallbladder cholesterosis, and drug-induced and toxic liver damage, wherein the active components - liver-protective merdication and prebiotic - taken in effective doses are mixed with microcrystalline cellulose, lactose, cornstarch, potato starch, hydroxypropyl methylcellulose, carboxymethylcellulose, oxypropyl methylcellulose, oxypropyl cellulose, with their pharmaceutically permissible salts, Ludipress, calcium stearate, magnesium stearate, polysorbate, polyvinylpyrrolidone, polyethylene glycol, talcum, titanium dioxide, or silicon dioxide.

## Revendications

1. Composition pharmaceutique destinée à une utilisation dans le traitement et la prévention de la récidive de maladies hépatiques causées par une perturbation du métabolisme du cholestérol, choisies dans le groupe suivant : les maladies de la vésicule biliaire, surtout avec calculs cholestériques, la stéatohépatite alcoolique et non alcoolique, la cirrhose du foie biliaire primaire, la cholestérose de la vésicule biliaire, les lésions hépatiques causées par des médicaments et toxiques, la composition pharmaceutique étant utilisée de manière interne et comprenant en tant que substances actives un médicament hépatoprotecteur choisi dans le groupe suivant : les acides choliques, les acides chénodésoxycholiques, les acides désoxycholiques, les acides ursodésoxycholiques, les acides lithocholiques, les acides tauroursodésoxycholiques, les acides glycodésoxycholiques, les acides taurocholiques, les acides glycocholiques et la lactulose en tant que prébiotique, qui sont choisis en doses efficaces.

2. Composition pharmaceutique destinée à une utilisation selon la revendication 1, **caractérisée en ce qu'**elle est utilisée de manière interne en tant que complément alimentaire.

3. Composition pharmaceutique destinée à une utilisation selon la revendication 1 ou 2, **caractérisée en ce qu'**elle comprend en tant que médicament hépatoprotecteur de l'acide biliaire ou un sel de l'acide biliaire et un prébiotique en un rapport de 1:2 à 1:250 selon la masse de substance pure.

4. Composition pharmaceutique destinée à une utilisation selon la revendication 1, **caractérisée en ce qu'**elle comprend en tant que médicament hépatoprotecteur des phospholipides essentiels choisis dans le groupe suivant : la phosphatidylcholine, la képhaline, le phosphatidylinositol.

5. Composition pharmaceutique destinée à une utilisation selon la revendication 1 ou 4, **caractérisée en ce qu'**elle comprend en tant que médicament hépatoprotecteur des phospholipides essentiels et un prébiotique en un rapport de 1:0,1 à 1:100 selon la masse de substance pure.

6. Composition pharmaceutique destinée à une utilisation selon la revendication 1, **caractérisée en ce qu'**elle comprend en outre de la cellulose microcristalline, du lactose, de l'amidon de maïs, de l'amidon, de l'hydroxypropylméthyleellulose, de la carboxyméthylcellulose, de l'oxypropylméthylcellulose, de l'oxypropylcellulose, leurs sels pharmaceutiquement autorisés, Ludipress, du stéarate de calcium, du stéarate de magnésium, du polysorbate, de la polyvinyipyrrolidone, du polyéthylène glycol, du talc, du dioxyde de titane ou du dioxyde de silicium.

7. Composition pharmaceutique destinée à une utilisation selon la revendication 1, **caractérisée en ce qu'**elle est configurée sous la forme de comprimés, de granules, d'ovules, de poudres, de gélules, de suspensions, de pâtes, de sirops, d'émulsions ou de gels, qui sont prévus pour une utilisation interne en doses correspondant aux doses connues des médicaments hépatoprotecteurs et des prébiotiques, 2 à 3 fois par jour.

8. Fabrication d'une composition pharmaceutique selon la revendication 1 pour le traitement de maladies hépatiques choisies dans le groupe suivant : les maladies de la vésicule biliaire, surtout avec calculs cholestériques, la stéatohépatite alcoolique et non alcoolique, la cirrhose du foie biliaire primaire, la cholestérose de la vésicule biliaire, les lésions hépatiques causées par des médicaments et toxiques, les composants actifs (médicament hépatoprotecteur et préblotique) pris en doses efficaces étant mélangés avec de la cellulose microcristalline, du lactose, de l'amidon de maïs, de l'amidon de pomme de terre, de l'hydroxypropylméthylcellulose, de la carboxyméthylcellulose, de l'oxypropylméthylcellulose, de l'oxypropylcellulose, leurs sels pharmaceutiquement autorisés, Ludipress, du stéarate de calcium, du stéarate de magnésium, du polysorbate, de la polyvinylpyrrolidone, du polyéthylène glycol, du talc, du dioxyde de titane ou du dioxyde de silicium.
